# EUROPÄISCHE PATENTSCHRIFT

(11) **EP 4 124 310 B1**
(45) Veröffentlichungstag und Bekanntmachung des Hinweises auf die Patenterteilung: **18.10.2023**
(21) Anmeldenummer: 21187788.1
(22) Anmeldetag: 26.07.2021
(51) Int. Cl.: A61B 18/12, A61B 18/00

(54) **GENERATOR MIT RÜCKSPEISEEINRICHTUNG**
GENERATOR WITH FEEDBACK DEVICE
GÉNÉRATEUR POURVU DE DISPOSITIF DE RÉCUPÉRATION

(43) Veröffentlichungstag der Anmeldung: 01.02.2023
(73) Patentinhaber: Erbe Elektromedizin GmbH, 72072 Tübingen (DE)
(72) Erfinder: Ripplinger, Thomas, 72072 Tübingen (DE)
(74) Vertreter: Rüger Abel Patentanwälte PartGmbB

(56) Entgegenhaltungen:
- EP-B1- 1 499 254
- DE-C2- 10 046 592
- US-A1- 2014 018 795
- US-A1- 2018 042 660

## Beschreibung

Die Erfindung betrifft einen elektrochirurgischen Generator mit einer Rückspeiseeinrichtung.

Elektrochirurgische Generatoren stellen typischerweise hochfrequente elektrische Spannung zum Betrieb eines elektrischen Instruments bereit. Solche Generatoren enthalten meist einen Schwingkreis, der die hochfrequente elektrische Spannung und den hochfrequenten elektrischen Strom zur Speisung des chirurgischen Instruments bereitstellt. In vielen Fällen muss die Hochfrequenzspannung moduliert werden, um bestimmte chirurgische Zwecke zu erreichen.

Aus der DE 100 46 592 A1 ist ein solcher Generator bekannt, bei dem eine hochfrequente elektrische Spannung mit einer Rechteckwelle amplitudenmoduliert wird. Am Ende eines Schwingungszyklus des Schwingkreises muss die Schwingung möglichst schnell gestoppt werden. Dazu sieht die DE 100 46 592 A1 einen Dämpfungsschaltkreis vor. Dieser enthält eine transformatorisch mit der Schwingkreisspule des Generators gekoppelte Spule, die dem Schwingkreis entnommene Energie einem Dämpfungswiderstand zuführt, der die Energie in Wärme umsetzt. Dadurch wird das Ausschwingen des Generatorschwingkreises beschleunigt und die Schwingung schnell gestoppt.

Zwar wird mit diesem Verfahren eine gute Modulation der chirurgischen Spannung erreicht, jedoch geht damit auch ein hoher Energieverbrauch einher.

Zur Abhilfe schlägt die EP 2 424 458 B1 vor, anstelle eines Widerstands zur Vernichtung der im Schwingkreis enthaltenen Energie eine Rückspeiseschaltung vorzusehen, die die Energie zu Ende eines HF-Impulses auf einen Speicherkondensator zurückleitet und lediglich einen Energierest, der nicht mehr auf den Speicherkondensator zurückleitbar ist, in einem ohmschen Widerstand im Wärme umsetzt. Damit wird der Prozess des Stoppens der Schwingung des Schwingkreises in zwei Phasen durchgeführt. In einer ersten Phase wird Energie vom Schwingkreis auf einen Speicherkondensator zurückgespeist und in einer zweiten Phase wird im Schwingkreis noch enthaltene Restenergie in Wärme umgesetzt. Mit diesem Konzept ist eine höhere Energieeffizienz zu erreichen. Dennoch müssen noch immer Energieanteile vernichtet werden. Außerdem besteht der Wunsch nach einer effizienteren und schnelleren Dämpfung des Schwingkreises.

Weiterer Stand der Technik wird durch die DE 40 09 819 A1**,** die US 44 29 694**,** die JP H 08 - 2 99 356**,** die WO 98/07378 A1**,** die WO 03/090635**,** die US 4 281 373 A**,** die WO 98/27880**,** die DE 10 046 592 A1 und die US 6 261 286 B1.

Davon ausgehend ist es Aufgabe der Erfindung, einen Generator mit verbesserter Rückspeiseschaltung anzugeben.

Diese Aufgabe wird mit dem Generator nach Anspruch 1 gelöst:

Der erfindungsgemäße Generator weist eine Rückspeiseschaltung auf, die gesteuert aktivierbar und deaktivierbar ist. Zur gesteuerten Aktivierung und Deaktivierung dient z.B. ein entsprechendes Schaltelement. Die Rückspeiseschaltung verbindet, wenn sie aktiviert ist, den Schwingkreis des elektrochirurgischen Generators mit einem Pufferkondensator, um Energie aus dem Schwingkreis in die Versorgungsschaltung und insbesondere auf den dort vorgesehenen Pufferkondensator zurück zu speisen. Erfindungsgemäß enthält die Rückspeiseschaltung eine Spannungsvervielfacherschaltung, insbesondere eine Spannungsvervielfacherschaltung, die durch eine Kondensator-Dioden-Kombination gebildet ist. Die Spannungsvervielfacherschaltung entzieht, wenn sie aktiviert wird, dem Schwingkreis elektrische Energie und baut dabei mit jeder Schwingung ansteigend eine Spannung auf, die ein Vielfaches der dem Schwingkreis entnommenen Spannung ist. Die Größe der Spannungsvervielfachung ergibt sich aus der Anzahl der Stufen der Spannungsvervielfacherschaltung. Durch die Spannungsvervielfachung wird die im Schwingkreis vorhandene Energie sehr schnell und effizient auf den Pufferkondensator rückgespeist, wobei die Bedämpfung des Schwingkreises auch dann noch anhält, wenn die Spannung im Schwingkreis geringer ist als die Speisespannung. Außerdem kann die Spannung auf dem Pufferkondensator beim Rückspeisen ansteigen, ohne den Rückspeisevorgang zu behindern.

Insbesondere beseitigt das erfindungsgemäße Konzept die Notwendigkeit eines Dämpfungswiderstands zum Dämpfen von Restspannungen an dem Schwingkreis. Damit ermöglicht der erfindungsgemäße Generator einen Betrieb, bei dem die hochfrequente Spannung des Schwingkreises, die im Bereich von 100 kHz bis mehreren MHz liegen kann, mit hohen Modulationsfrequenzen im Bereich einiger 10 kHz, zum Beispiel 20 kHz, 40 kHz, 60 kHz, 80 kHz oder auch darüber. Außerdem wird insbesondere die Energieeffizienz von Moden verbessert, in denen die Einschaltdauer des HF-Impulses jeweils sehr kurz ist wie beispielsweise bei Schneidmoden, bei denen das Puls-Pause-Verhältnis der modulierten HF-Spannung besonders gering ist

Der Schwingkreis ist typischerweise ein Parallelschwingkreis, der aus mindestens einer Schwingkreisspule und mindestens einem Schwingkreiskondensator besteht. Dem Schwingkreis ist eine Erregerschaltung zugeordnet, die dazu eingerichtet ist, den Schwingkreis auf seiner Resonanzfrequenz zu erregen. Im einfachsten Fall enthält die Erregerschaltung einen gesteuerten Schalter, der dem Parallelschwingkreis impulsweise Energie zuführt. Die Erregerschaltung kann jedoch auch mehrere gesteuerte Schalter zum Beispiel in Halbbrückenschaltung oder in Brückenschaltung umfassen.

Die Versorgungsschaltung ist vorzugsweise eine Gleichspannungsquelle, die einen an ihrem Ausgang angeordneten Speicherkondensator aufweist. Die Gleichspannungsquelle kann eine Spannungswandlerschaltung sein, wie beispielsweise eine PFC-Schaltung (Power Factor Corretion Schaltung). Als solche kann ein Sperrwandler dienen. Die PFC-Schaltung stellt aus einer pulsierenden gleichgerichteten Netzwechselspannung eine zeitlich im Wesentlichen konstante Gleichspannung bereit. Andere Wandlerschaltungen können ebenso Anwendung finden.

Der am Ausgang der Versorgungsschaltung angeordnete Speicherkondensator bildet einen Puffer, der von der Rückspeiseschaltung gelieferte Energie aufnimmt. Diese steht dann wieder zur Speisung des Schwingkreises zur Verfügung.

Die Rückspeiseschaltung kann direkt mit der Schwingkreisspule verbunden sein. Alternativ kann die Rückspeiseschaltung mit einer Rückspeisespule verbunden sein, die mit der Schwingkreisspule transformatorisch koppelt. In beiden Fällen bewirkt die Spannungsvervielfacherschaltung eine Erhöhung der aus dem Schwingkreis abgeleiteten Spannung und somit eine effiziente Energierückspeisung.

Vorzugsweise ist die Spannungsvervielfacherschaltung eine sogenannte Kondensatorkaskade, insbesondere eine mehrstufige Kaskade. Eine solche Schaltung umfasst zwei Reihenschaltungen jeweils mehrerer Kondensatoren, wobei die Verbindungspunkte der Kondensatoren jeder Reihenschaltung über eine im Zickzack angeordnete Diodenkette mit den Verbindungspunkten der jeweils anderen Reihenschaltung von Kondensatoren verbunden sind. Zur Aktivierung oder Deaktivierung der Spannungsvervielfacherschaltung ist vorzugsweise ein Schalter vorgesehen. Dieser ist vorzugsweise zwischen der Rückspeisespule oder dem Schwingkreiskondensator und der Spannungsvervielfacherschaltung angeordnet. Dieses Konzept minimiert die kapazitive Kopplung zwischen der Rückspeiseschaltung und dem Schwingkreis bei inaktiver Rückspeiseschaltung und ermöglicht somit ungestörten Betrieb des Generators außerhalb der Rückspeisephasen. Der Schalter ist vorzugsweise dazu eingerichtet und so angesteuert, dass die Rückspeiseschaltung nur während der Rückspeisephasen mit dem Schwingkreis verbunden ist.

Der erfindungsgemäße Generator weist vorzugsweise eine Steuerschaltung auf, die dazu eingerichtet ist, die Erregerschaltung einerseits und die Rückspeiseschaltung andererseits abwechselnd zu aktivieren. Damit kann die Schwingung des Schwingkreises ein- und ausgeschaltet werden, sodass eine nahezu perfekte Rechteckmodulation der HF-Ausgangsspannung des Generators möglich wird. Andere Modulationsarten, z.B. Sägezahnmodulation sind möglich. Die Erfindung hat immer dann Vorteile, wenn die gewünschte Modulation steile Rückflanken, d.h. einen schnellen Stopp der HF-Schwingung erfordert.

Weitere Einzelheiten vorteilhafter Ausführungsformen sind Gegenstand von Unteransprüchen sowie der Beschreibung und der zugehörigen Zeichnung. Es zeigen:
Figur 1 den erfindungsgemäßen Generator mit angeschlossenem Instrument, in schematisierter Übersichtsdarstellung,
Figur 2 ein vereinfachtes Schaltbild des Generators nach Figur 1,
Figur 3 ein Schaltbild eines abgewandelten Generators gemäß Figur 1,
Figur 4 ein weiteres auszugsweises und schematisiertes Schaltbild eines Generators gemäß Figur 1 und
Figur 5 ein Diagramm zur Veranschaulichung des Schwingungsverhaltens der Generatoren nach Figur 1 bis 4.

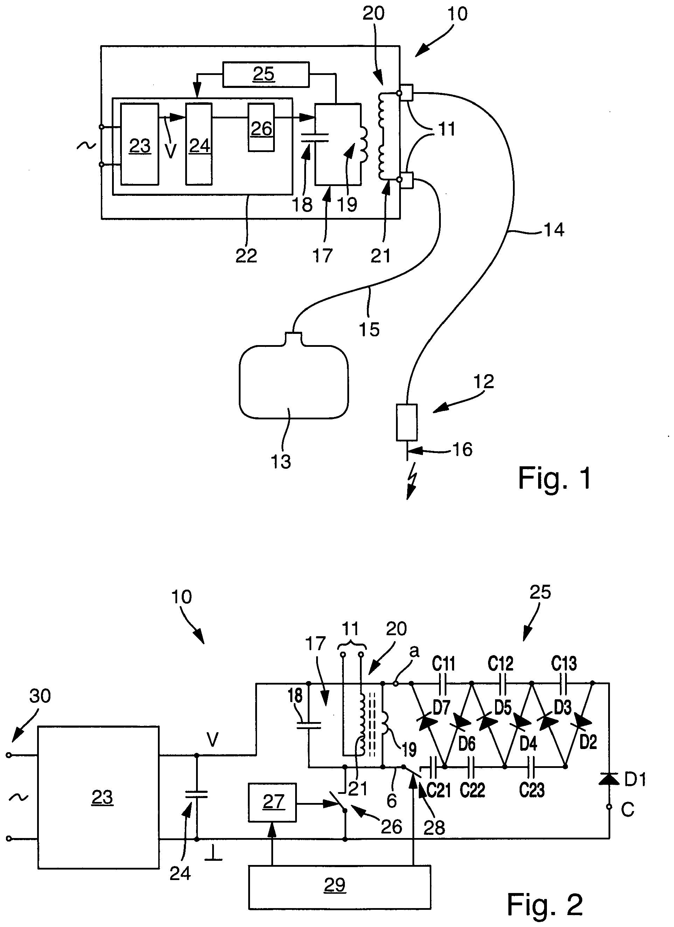

Figur 1 veranschaulicht einen Generator 10 zur Bereitstellung einer chirurgischen Spannung an einem Ausgang 11. An diesen kann ein bipolares chirurgisches Instrument oder, wie Figur 1 veranschaulicht, ein monopolares chirurgisches Instrument sowie eine Neutralelektrode 13 angeschlossen werden. Sowohl das Instrument 12 als auch die Neutralelektrode 13 können dazu durch entsprechende Leitungen 14, 15 mit dem Ausgang 11 verbunden sein.

Das Instrument 12 weist mindestens eine Elektrode 16 auf, mit der auf biologisches Gewebe des Patienten einzuwirken ist. Hingegen ist die Neutralelektrode 13 großflächig ausgebildet, um einen Stromübertritt zwischen dem Patienten und der Neutralelektrode 13 ohne physiologischen Effekt zu ermöglichen.

In Figur 1 ist das Instrument 12 schematisch veranschaulicht. Es kann sich um ein Schneidinstrument, ein Koagulationsinstrument sowie um jedes andere monopolare oder bipolare elektrochirurgische Instrument handeln. Im Falle eines bipolaren Instruments weist dieses zwei Elektroden auf, die beide über entsprechende Leitungen oder ein Kabel mit dem Ausgang 11 verbunden sind.

Der Generator 10 eignet sich insbesondere zur Versorgung von Instrumenten, die mit gepulster elektrischer Hochfrequenzspannung zu versorgen sind. Unter einer gepulsten elektrischen Hochfrequenzspannung (HF-Spannung) werden insbesondere Spannungen verstanden, die eine Grundfrequenz zwischen 100 kHz und 5 MHz vorzugsweise 300 kHz bis 500 kHz haben und die mit einer Rechteckimpulsfolge amplitudenmoduliert sind. Dies bedeutet, dass die Amplitude der von dem Generator 10 erzeugten HF-Spannung mit der Frequenz der Rechteckimpulsfolge ihren Wert zwischen einem ersten Wert und einem zweiten Wert wechselt, beispielsweise zwischen mehreren 100 V und 0 Volt oder zwischen mehreren 100 V und lediglich mehreren 10 V. Die HF-Spannung ist somit beispielsweise eine "Ein/Aus-gestastete" Spannung. Die Erfindung eignet sich aber auch zur Erzeugung einer HF-Spannung mit anderen Modulationsformen, beispielsweise HF-Spannung mit Sägezahnmodulation und allen anderen Modulationsformen, insbesondere solchen, bei denen es darauf ankommt, dass die HF-Schwingung am Ende eines HF-Spannungsimpulses schnell stoppt.

Die Struktur des Generators 10 ist in Figur 1 übersichtsmäßig veranschaulicht. Zur Erzeugung der gewünschten hochfrequenten Wechselspannung dient ein Schwingkreis 17, der mindestens einen Schwingkreiskondensator 18 und mindestens eine Schwingkreisspule 19 umfasst, die zueinander parallel geschaltet sind.

Um dem Instrument 12 elektrische Hochfrequenzenergie zuzuleiten, ist der Schwingkreis 17 mit einer Auskoppelschaltung 20 verbunden, die im vorliegenden Ausführungsbeispiel durch mindestens eine magnetisch (transformatorisch) mit der Schwingkreisspule 19 koppelnde Auskoppelspule 21 gebildet ist. Andere Auskoppelschaltungen sind möglich. Die Auskoppelspule 21 kann aus mehreren Teilspulen bestehen, die miteinander in Reihe geschaltet sind. Vorzugsweise bilden die Schwingkreisspule 19 und die Auskoppelspule 21 somit einen Transformator mit einem Übertragungsfaktor größer als 1. Der Übertragungsfaktor ist das Verhältnis der Windungszahl der Auskoppelspule 21 zu der Windungszahl der Schwingkreisspule 19.

Zur Anregung und zum Unterhalt einer Schwingung in dem Schwingkreise 17 ist eine Erregerschaltung 22 vorgesehen, die dem Schwingkreis 17 Anregungsenergie zuleitet. Die Erregerschaltung 22 erhält von einer Gleichspannungsquelle 23 zur Bereitstellung von Gleichspannungsleistung. Eine entsprechende Gleichspannung V führende Leitung ist mit einem Pufferkondensator 24 verbunden. Dieser dient der Energiespeicherung und auch der Aufnahme von Energie, die beim Stoppen der HF-Schwingung des Schwingkreises 17 aus diesem zurück geleitet wird.

Zur Rückgewinnung von Energie aus dem Schwingkreis 17 beim Stoppen der HF-Schwingung ist eine Rückspeiseschaltung 25 vorgesehen. Diese verbindet den Schwingkreis 17 mit dem Pufferkondesator 24, um in dem Schwingkreis 17 vorhandene Energie immer dann auf den Pufferkondensator 24 zurückzuführen, wenn die Schwingung des Schwingkreises 17 möglichst schnell stoppen soll.

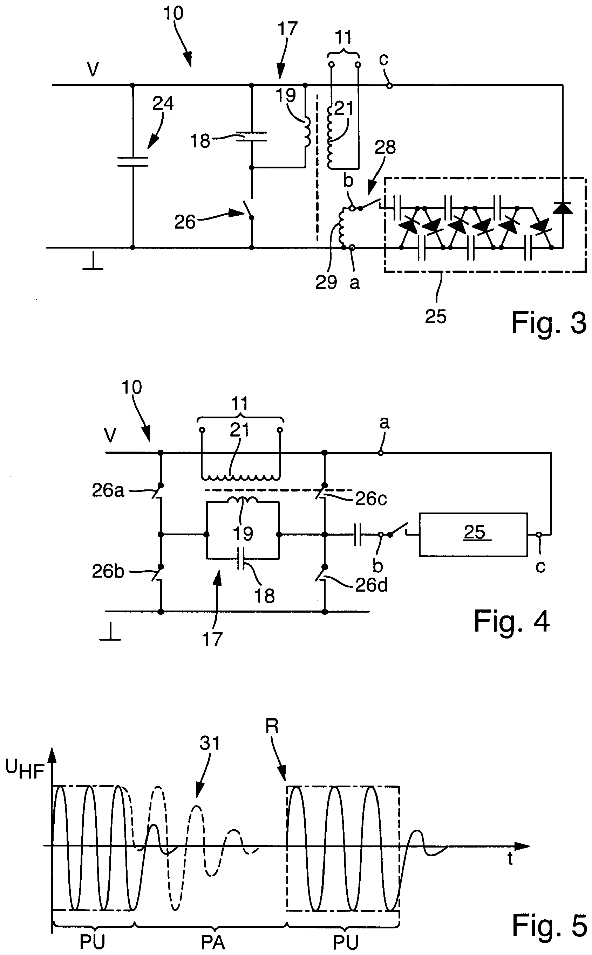

Figur 2 veranschaulicht den Schaltplan des Generators 10 insbesondere auch der Rückspeiseschaltung 25 detaillierter. Wie ersichtlich, kann die Erregerschaltung 22 im einfachsten Fall durch einen elektronisch gesteuerten Schalter 26 und eine ihm zugehörige Ansteuerschaltung 27 gebildet sein. Der Schalter 26 ist zwischen dem Schwingkreis 17 oder einer Rückspeisespule 29 (Figur3) und einem Eingang der Rückspeiseschaltung 25 angeordnet. Leitet er, ist die Rückspeiseschaltung25 aktiv und führt Energie aus dem Schwingkreis 17 unter Bedämpfung desselben auf den Pufferkondensator 24 zurück. Sperrt er, ist die Rückspeiseschaltung inaktiv und bedämpft den Schwingkreis 17 nicht.

Die Rückspeiseschaltung 25 kann direkt an den Schwingkreis 17 angeschlossen sein. Dabei können ihre beiden Eingangsleitungen a, b unmittelbar mit dem Eingang der Rückspeiseschaltung verbunden sein. Leitungen a und c bilden dabei den Ausgang der Rückspeiseschaltung 25.

Die Rückspeiseschaltung 25 ist vorzugsweise eine Spannungsvervielfacherschaltung. Diese umfasst zwei Stränge mit Reihenschaltungen von jeweils mehreren beispielsweise 2, 3 oder 4 oder mehreren Kondensatoren. In einem Strang sind Kondensatoren C11, C12, C13 miteinander in Reihe geschaltet. In dem anderen parallel dazu verlaufenden Strang sind Kondensatoren C21, C21, C23 in gleicher Anzahl mit einander in Reihe geschaltet. Die Verbindungspunkte zwischen den jeweiligen Kondensatoren der beiden Stränge sind durch Dioden untereinander verbunden, sodass sich eine übliche Spannungsvervielfacherschaltung bildet. Die Dioden D1 bis D7 sind zwischen den beiden aus den Kondensatoren C11 bis C13 und C21 bis C23 gebildeten Strängen im Zickzack angeordnet. Die Dioden D1 bis D7 sind in gleicher Polung hintereinander geschaltet, d.h. an jedem Verbindungspunkt treffen sich die Anode einer Diode mit der Kathode der anderen Diode.

Zu der Rückspeiseschaltung 25 gehört ein Aktivierungsschalter 28, der in der Leitung b angeordnet ist. Die Leitung b bildet eine Verbindung zwischen dem Schwingkreis 17 und der Rückspeiseschaltung 25. Der Aktivierungsschalter ist dazu eingerichtet, in der Leitung b einen Strompfad zu öffnen und zu schließen.

Der Aktivierungsschalter 28 und der elektronische Schalter 26 sind in aufeinander abgestimmter Weise gesteuert. Zur Steuerung kann eine Steuereinrichtung 29 vorgesehen sein, die die Schalter 26, 28 direkt oder auch unter Zwischenschaltung entsprechender Ansteuerschaltungen, wie zum Beispiel der Ansteuerschaltung 27, steuert. Die Steuerschaltung öffnet und schließt den Schalter 26 im Takt der Schwingung des Schwingkreises 17, solange dieser erregt sein soll. Soll die Schwingung gestoppt werden, bleibt der Schalter 26 in nichtleitendem Zustand und der Schalter 28 wird in den leitenden Zustand überführt. Soll der Schwingkreis wieder beginnen zu schwingen, wird der Schalter 29 in nichtleitenden Zustand überführt und der Schalter 26 wird mit der der Resonanzfrequenz des Schwingkreises 17 entsprechenden Schaltfrequenz wieder ein- und ausgeschaltet.

Eine abgewandelte Ausführungsform des Generators 10 ist in Figur 3 veranschaulicht. Hinsichtlich der grundsätzlichen Beschreibung gelten die vorstehenden Ausführungen unter Zugrundelegung der bereits eingeführten Bezugszeichen entsprechend:

Während die Leitungen a und b der Rückspeiseschaltung 25 bei der Ausführungsform nach Figur 2 direkt mit dem Schwingkreis verbunden sind, sind die Leitungen a und b bei der Ausführungsform nach Figur 3 mit einer Rückspeisespule 29 verbunden, die mit der Schwingkreisspule 19 transformatorisch gekoppelt ist. Die Rückspeisespule 29 und die Schwingkreisspule 19 bilden somit einen Transformator, dessen Übertragungsfaktor vorzugsweise zwischen 1 und 2 liegt. Andere Übertragungsfaktoren sind möglich. Der Übertragungsfaktoren definiert sich als das Verhältnis der Windungszahl der Auskoppelspule 29 zu der Windungszahl der Schwingkreisspule 19.

Figur 4 veranschaulicht eine weitere Abwandlung des Generators 10. Bei diesem umfasst die Erregerschaltung zur Anregung des Schwingkreises 17 mehrere Schalter 26a, 26b, 26c, 26d, die eine Brückenschaltung bilden, um den Schwingkreis 17 anzuregen. Figur 4 veranschaulicht eine Vollbrückenschaltung mit vier Schalter 26a bis 26d. Es kann zum Anregen des Schwingkreises 17 jedoch auch eine Halbbrückenschaltung Anwendung finden, bei der zwei Schalter, beispielsweise die Schalter 26a, 26b, durch Kondensatoren ersetzt sind.

Der insoweit beschriebene Generator 10 nach Figur 2 arbeitet wie folgt:
Zur Funktionsbeschreibung wird rein beispielhaft vorausgesetzt, dass der Schwingkreis 17 eine Resonanzfrequenz von zwischen 200 kHz und 1 MHz beispielsweise 350 kHz, 500 kHz oder dergleichen aufweist. Entsprechend ist die Steuereinrichtung 29 dazu vorgesehen, den Schalter 26 mit dieser Frequenz zu öffnen und zu schließen, um den Schwingkreis 17 auf seiner Resonanzfrequenz zu erregen. Die zum Beispiel aus einem allgemeinen Versorgungsnetz 30 gespeiste Gleichspannungsquelle 23 gibt eine Gleichspannung von zum Beispiel mehreren 100 V, beispielsweise 300 V zwischen Masse und der Betriebsspannungsleitung V ab, sodass der Pufferkondensator 24 mit Betriebsspannung (beispielsweise 300 V) geladen ist.

Wird nun angenommen, dass die in dem Schwingkreis 17 vorhandene Schwingung mit einer Rechteckfunktion R wie in Figur 5 veranschaulicht gepulst werden soll, müssen zwischen einzelnen Pulsen PU Schwingpausen PA erzeugt werden. Die Pulse PU der HF-Spannung U_{HF} können beispielsweise einige ps, zum Beispiel 5 ps, lang sein. Die zeitliche Länge der Pausen PA zwischen einzelnen Impulsen PU der HF-Spannung hängt von der Modulationsfrequenz und dem sogenannten Tastverhältnis ab. Insbesondere bei Schneidmoden, bei denen die HF-Spannung U_{HF} sehr hoch (einige 1000 V) sein kann und dafür die Impulse PU der HF-Spannung sehr kurz sind (ein bis wenige HF-Schwingungen pro Impuls PU der HF-Spannung) können die Puls-Pause-Verhältnisse sehr klein und somit die Dauer der HF-Pause PA relativ groß sein (z.B. 100 ps).

Bei dem Generator 10 kommt es darauf an, die HF-Schwingung am Ende jedes HF-Spannungsimpulses PU möglichst schnell und effizient zu stoppen, sodass kein oder nur wenig Nachschwingen des Schwingkreises 17 auftritt. Figur 5 veranschaulicht eine Ausschwingkurve 31 gestrichelt, wie sie an einem Generator ohne Bedämpfung durch die Rückspeiseschaltung 25 auftreten kann. Weil aber die Steuereinrichtung 29 am Ende eines Impulses PU der HF-Spannung den Schalter 28 schließt und damit die Rückspeiseschaltung 25 aktiviert, wir der in Figur 5 dick dargestellte Ausschwingvorgang drastisch gekürzt. Dabei wird davon ausgegangen, dass die Rückspeiseschaltung 25 von vorherigen Rückspeisungen her geladene Kondensatoren C11 bis C23 aufweist. Die in dem Schwingkreis 17 enthaltene Energie wird somit mit wenigen Schwingungen des Schwingkreises 17 auf den Pufferkondensator 24 übertragen. Auch mit abklingender Schwingungsamplitude des Schwingkreises 17 setzt sich der Rückspeisevorgang fort, weil die Rückspeiseschaltung 25 als Spannungsvervielfacherschaltung arbeitet und somit die beim Abklingen der HF-Schwingung absinkende Spannung durch Vervielfachung wieder auf einen zur Speisung des Pufferkondensators 24 ausreichenden Wert hebt.

Der erfindungsgemäße elektrochirurgische Generator 10 weist einen Schwingkreis auf, der durch eine Erregerschaltung mit einer vorzugsweise in der Nähe seiner Resonanzfrequenz liegendem Frequenz zur Schwingung erregt wird, wobei diese Schwingung periodisch unterbrochen werden soll. Dies beispielsweise im Rahmen einer Puls-Pause-Modulation der zur erzeugenden hochfrequenten Spannung U_{HF}, deren Grundfrequenz von beispielsweise 350 kHz oder 500 kHz mit einer Modulationsfrequenz von zum Beispiel 50 kHz moduliert werden kann. Andere Modulationsfrequenzen sind möglich. Typischerweise liegen sie unter 100 kHz. Um die Schwingung in dem Schwingkreis des Generators möglichst abrupt zu stoppen, ohne die in dem Schwingkreis 17 gespeicherte Energie zu verlieren, ist eine Rückspeiseschaltung 25 vorgesehen, die durch eine Spannungsvervielfacherschaltung gebildet ist. Diese hat gegenüber einer hochtransformierenden Koppelwicklung den Vorteil einer geringen kapazitiven Belastung des Schwingkreises 17, insbesondere weil sie lediglich in der Rückspeisephase über den Schalter 28 elektrisch wirksam mit dem Schwingkreis 17 verbunden ist. Dieses Konzept gestattet eine effiziente Energierückgewinnung und ermöglicht somit die präzise Amplitudenmodulation insbesondere Rechteckmodulation (Ein-Aus-Tasten) der hochfrequenten Spannung U_{HF}.

### Bezugszeichen:

- 10: Generator
- 11: Ausgang
- 12: Instrument
- 13: Neutralelektrode
- 14, 15: Leitungen
- 16: Elektrode
- 17: Schwingkreis
- 18: Schwingkreiskondensator
- 19: Schwingkreisspule
- 20: Auskoppelschaltung
- 21: Auskoppelspule
- 22: Versorgungsschaltung / Erregerschaltung
- 23: Gleichspannungsquelle
- 24: Pufferkondensator
- 25: Rückspeiseschaltung
- 26: elektronischer Schalter
- 27: Ansteuerschaltung
- a, b, c: Leitungen
- C11 - C13: Kondensatoren eines ersten Stranges
- C21 - C23: Kondensatoren eines zweiten Stranges
- D1 - D7: Dioden
- 28: Aktivierungsschalter
- 29: Steuereinrichtung
- 30: Versorgungsnetz
- PU: HF-Spannungsimpuls
- PA: HF-Pause
- 31: Ausschwingkurve
- U_{HF}: hochfrequente Spannung

## Patentansprüche

1. Elektrochirurgischer Generator (10)
mit einem Schwingkreis (17), der mindestens eine Schwingkreisspule (19) und mindestens einen Schwingkreiskondensator (18) aufweist und mit einer Erregerschaltung (22) verbunden ist, die dazu eingerichtet ist, in dem Schwingkreis (17) eine elektrische Schwingung zu erzeugen,
mit einer Gleichspannungsquelle (23), die zur Bereitstellung einer Speisespannung (V) für den Schwingkreis (17) eingerichtet ist und mit einem Pufferkondensator (24) verbunden ist,
mit einer Auskoppelschaltung (20), die einerseits mit dem Schwingkreis (17) und andererseits mit einer Anschlusseinrichtung (11) für ein chirurgisches Instrument (12) verbunden ist,
mit einer Rückspeiseschaltung (25), mittels derer in dem Schwingkreis (17) gespeicherte Energie auf den Pufferkondensator (24) rücküberführbar ist, **dadurch gekennzeichnet,**
**dass** die Rückspeiseschaltung (25) eine Spannungsvervielfacherschaltung (C11-C23, D1-D7) aufweist.

2. Generator nach Anspruch 1, wobei der Schwingkreis ein Parallelschwingkreis ist.

3. Generator nach einem der vorstehenden Ansprüche, wobei die Versorgungsschaltung (22) mindestens einen gesteuerten Schalter (26) umfasst.

4. Generator nach einem der vorstehenden Ansprüche, wobei die Versorgungsschaltung (22) eine Gleichspannungsquelle (23) mit einem an ihrem Ausgang angeordneten Speicherkondensator (24) aufweist.

5. Generator nach einem der vorstehenden Ansprüche, wobei die Auskoppelschaltung (20) durch eine Auskoppelspule (21) gebildet ist, die mit der Schwingkreisspule (19) transformatorisch gekoppelt ist.

6. Generator nach einem der vorstehenden Ansprüche, wobei die Rückspeiseschaltung (25) eine Rückspeisespule (29) aufweist, die mit der Schwingkreisspule (19) transformatorisch gekoppelt ist.

7. Generator nach einem der vorstehenden Ansprüche, wobei die Rückspeiseschaltung (25) mit der Schwingkreisspule (19) verbunden ist.

8. Generator nach einem der vorstehenden Ansprüche, wobei die Spannungsvervielfacherschaltung (C11-C23, D1-D7) eine Kondensatorkaskade ist.

9. Generator nach Anspruch 8, wobei die Kondensatorkaskade eine mehrstufige Kaskade ist.

10. Generator nach einem der Ansprüche 8 oder 9, wobei die Kondensatorkaskade zwei Reihenschaltungen (C11-C13; C21-C23) jeweils mehrerer Kondensatoren (C11-C23) aufweist, wobei jeder zwischen zwei Kondensatoren (C11/C12, C12/C13) einer Reihenschaltung (C11-C13) vorhandene Verbindungpunkt mit zwei antiparallel angeordneten Dioden (D3 - D6) verbunden ist, die mit unterschiedlichen Verbindungspunkten (C21/C22, C22/C23) der jeweils anderen Reihenschaltung (C21-C23) verbunden sind.

11. Generator nach Anspruch 6, wobei zwischen der Rückspeisespule (29) und der Spannungsvervielfacherschaltung ein Schalter (28) angeordnet ist.

12. Generator nach einem der Ansprüche 6 oder 11, wobei das Verhältnis der Windungszahl der Rückspeisespule (29) zu der Windungszahl der Schwingkreisspule (19) zwischen Zwei und Eins liegt.

13. Generator nach einem der vorstehenden Ansprüche, wobei der Generator (10) zusätzlich eine Steuerschaltung (29) umfasst, die dazu eingerichtet ist, die Erregerschaltung (2) und die Rückspeiseschaltung abwechselnd zu aktivieren.

14. Generator nach Anspruch 11, wobei der Schalter (28) ein Halbleiterschalter ist.

15. Generator nach Anspruch 14, wobei die Rückspeisespule (29) mit einem Ende mit Masse und mit ihrem andere Ende mit dem Schalter (28) verbunden ist.

## Claims

1. Electrosurgical generator (10)
having an oscillating circuit (17) that comprises at least one oscillating circuit inductor (19) and at least one oscillating circuit capacitor (18) and that is connected with an excitation circuit (22), which is configured to create an electrical oscillation in the oscillating circuit (17),
having a direct voltage source (23) that is configured for providing a supply voltage (V) for the oscillating circuit (17) and that is connected with a buffer capacitor (24),
having a decoupling circuit (20) that is connected with the oscillating circuit (17) on one hand and with a connection device (11) for a surgical instrument (12) on the other hand,
having a regeneration circuit (25) by means of which energy stored in the oscillating circuit (17) can be transferred back to the buffer capacitor (24),
**characterized in that** the regeneration circuit (25) comprises a voltage multiplier circuit (C11-C23, D1-D7).

2. Generator according to claim 1, wherein the oscillating circuit is a parallel oscillating circuit.

3. Generator according to any of the preceding claims, wherein the supply circuit (22) comprises at least one controlled switch (26).

4. Generator according to any of the preceding claims, wherein the supply circuit (22) comprises a direct voltage source (23) having a buffer capacitor (24) arranged at its output.

5. Generator according to any of the preceding claims, wherein the decoupling circuit (20) is realized by a decoupling inductor (21) that is coupled with the oscillating circuit inductor (19) in a transformer-type manner.

6. Generator according to any of the preceding claims, wherein the regeneration circuit (25) comprises a regeneration inductor (29) that is coupled with the oscillating circuit inductor (19) in a transformer-type manner.

7. Generator according to any of the preceding claims, wherein the regeneration circuit (25) is connected with the oscillating circuit inductor (19).

8. Generator according to any of the preceding claims, wherein the voltage multiplier circuit (C11-C23, D1-D7) is a capacitor cascade.

9. Generator according to claim 8, wherein the capacitor cascade is a multiple stage cascade.

10. Generator according to claim 8 or 9, wherein the capacitor cascade comprises two series connections (C11-C13; C21-C23) of multiple capacitors (C11-C23) respectively, wherein each connection point that is present between two capacitors (C11/C12, C12/C13) of one series connection (C11-C13) is connected with two diodes (D3-D6) arranged anti-parallel that are connected with different connection points (C21/C22, C22/C23) of the respective other series connections (C21-C23).

11. Generator according to claim 6, wherein a switch (28) is arranged between the regeneration inductor (29) and the voltage multiplier circuit.

12. Generator according to any of the claims 6 or 11, wherein the ratio of the number of windings of the regeneration inductor (29) relative to the number of windings of the oscillating circuit inductor (19) is between two and one.

13. Generator according to any of the preceding claims, wherein the generator (10) comprises in addition a control device (29) that is configured to alternatingly activate the excitation circuit (22) and the regeneration circuit.

14. Generator according to claim 11, wherein the switch (28) is a semi-conductor switch.

15. Generator according to claim 14, wherein the regeneration inductor (29) is connected with one end to ground and with its other end to the switch (28).

## Revendications

1. Générateur électrochirurgical (10),
comprenant un circuit oscillant (17) qui présente au moins une bobine de circuit oscillant (19) et au moins un condensateur de circuit oscillant (18) et est relié à un circuit d'excitation (22) qui est conçu pour générer une oscillation électrique dans le circuit oscillant (17),
comprenant une source de tension continue (23) qui est conçue pour la mise à disposition d'une tension d'alimentation (V) pour le circuit oscillant (17) et est reliée à un condensateur tampon (24),
comprenant un circuit de découplage (20) qui est relié d'un côté au circuit oscillant (17) et de l'autre côté à un dispositif de raccordement (11) pour un instrument chirurgical (12),
comprenant un circuit de récupération (25) qui permet de renvoyer au condensateur tampon (24) de l'énergie stockée dans le circuit oscillant (17),
**caractérisé en ce que**
le circuit de récupération (25) présente un circuit multiplicateur de tension (C11-C23, D1-D7).

2. Générateur selon la revendication 1, dans lequel le circuit oscillant est un circuit oscillant parallèle.

3. Générateur selon l'une des revendications précédentes, dans lequel le circuit d'alimentation (22) comprend au moins un interrupteur (26) commandé.

4. Générateur selon l'une des revendications précédentes, dans lequel le circuit d'alimentation (22) présente une source de tension continue (23) comportant un condensateur de stockage (24) disposé à sa sortie.

5. Générateur selon l'une des revendications précédentes, dans lequel le circuit de découplage (20) est constitué d'une bobine de découplage (21) qui est couplée par transformation à la bobine de circuit oscillant (19).

6. Générateur selon l'une des revendications précédentes, dans lequel le circuit de récupération (25) présente une bobine de récupération (29) qui est couplée par transformation à la bobine de circuit oscillant (19).

7. Générateur selon l'une des revendications précédentes, dans lequel le circuit de récupération (25) est relié à la bobine de circuit oscillant (19).

8. Générateur selon l'une des revendications précédentes, dans lequel le circuit multiplicateur de tension (C11-C23, D1-D7) est une cascade de condensateurs.

9. Générateur selon la revendication 8, dans lequel la cascade de condensateurs est une cascade à plusieurs étages.

10. Générateur selon l'une des revendications 8 ou 9, dans lequel la cascade de condensateurs présente deux montages en série (C11-C13 ; C21-C23) respectivement de plusieurs condensateurs (C11-C23), sachant que chaque point de liaison existant entre deux condensateurs (C11/C12, C12/C13) d'un montage en série (C11-C13) est relié à deux diodes (D3 - D6) qui sont montées en antiparallèle et sont reliées à des points de liaison (C21/C22, C22/C23) différents, respectivement de l'autre montage en série (C21-C23).

11. Générateur selon la revendication 6, dans lequel un interrupteur (28) est placé entre la bobine de récupération (29) et le circuit multiplicateur de tension.

12. Générateur selon l'une des revendications 6 ou 11, dans lequel le rapport du nombre d'enroulements de la bobine de récupération (29) au nombre d'enroulements de la bobine du circuit oscillant (19) est compris entre deux et un.

13. Générateur selon l'une des revendications précédentes, dans lequel le générateur (10) comprend en outre un circuit de commande (29) qui est conçu pour activer en alternance le circuit d'excitation (2) et le circuit de récupération.

14. Générateur selon la revendication 11, dans lequel l'interrupteur (28) est un interrupteur semi-conducteur.

15. Générateur selon la revendication 14, dans lequel la bobine de récupération (29) est reliée par une extrémité à la masse et par son autre extrémité à l'interrupteur (28).
